# EUROPEAN PATENT SPECIFICATION

(11) **EP 3 630 151 B1**
(45) Date of publication and mention of the grant of the patent: **17.12.2025**
(21) Application number: 18808988.2
(22) Date of filing: 29.05.2018
(51) Int. Cl.: A61K 31/125, A61K 33/00, A61K 35/51, C12N 5/071, A61K 47/42

(54) **VISIBLE LIGHT POLYMERIZATION OF POLYETHYLENE GLYCOL (PEG) HYDROGELS**
POLYMERISATION DURCH SICHTBARES LICHT VON POLYETHYLENGLYKOL(PEG)-HYDROGELEN
POLYMÉRISATION PAR LUMIÈRE VISIBLE D'HYDROGELS DE POLYÉTHYLÈNE GLYCOL (PEG)

(30) Priority: 30.05.2017 US 201762512337 P
(43) Date of publication of application: 08.04.2020
(73) Proprietor: Stem Pharm, Incorporated, Madison, Wisconsin 53711 (US)
(72) Inventor: KHALIL, Andrew, Madison, Wisconsin 53711 (US); STECHMANN LEBAKKEN, Connie, Madison, Wisconsin 53711 (US); MURPHY, William L., Madison, Wisconsin 53711 (US)
(74) Representative: Carpmaels & Ransford LLP
(86) International application number: PCT/US2018/034928
(87) International publication number: WO 2018/222611

(56) References cited:
- WO-A2-00/56375
- US-A1- 2014 288 022
- US-A1- 2015 291 929
- US-A1- 2015 291 929
- US-A1- 2016 129 149
- US-A1- 2016 175 800
- CHIEN-CHI LIN ET AL: "Thiol-norbornene photoclick hydrogels for tissue engineering applications", JOURNAL OF APPLIED POLYMER SCIENCE, vol. 132, no. 8, 18 October 2014 (2014-10-18), US, pages 41563 - 1, XP055387302, ISSN: 0021-8995, DOI: 10.1002/app.41563
- DIAS ET AL.: "Microcarriers with Synthetic Hydrogel Surfaces for Stem Cell Expansion", ADVANCED HEALTHCARE MATERIALS, vol. 6, no. 16, 16 May 2017 (2017-05-16), pages 1 - 9, XP055553129
- GUO ET AL.: "Effect of initiator on photopolymerization of acidic, aqueous dental model adhesives", JOURNAL OF BIOMEDICAL MATERIALS RESEARCH. PART A, vol. 90A, no. 4, 15 September 2009 (2009-09-15), pages 1120 - 1127, XP055553136
- LEIGHT ET AL.: "Direct measurement of matrix metalloproteinase activity in 3D cellular microenvironments using a fluorogenic peptide substrate", BIOMATERIALS, vol. 34, no. 30, 2 July 2013 (2013-07-02), pages 7344 - 7352, XP028679357

## Description

This application claims priority to United States provisional patent application serial number 62/512,337, filed May 30, 2017.

### FIELD OF THE DISCLOSURE

Provided herein are compositions and methods for generating visible light photopolymerized hydrogels to support cell viability, expansion, and encapsulation.

### BACKGROUND

Tris(bipyridine)ruthenium(II) chloride [Ru(bpy)₃]Cl₂ is a transition metal complex that serves the role of lowering the activation energy and subsequent radical transfer of the phenolic side chains of tyrosine residues in proteins. Coupled with radical co-initiators (fast and slow), Tris(bipyridine)ruthenium(II) chloride [Ru(bpy)₃]Cl₂ may be used to activate phenolic residues of tissue culture treated polystyrene for covalent coupling in radical-initiated photocross-linking methods of polyethylene glycol (PEG)-based hydrogels. Co-initiators include persulfates (fast co-initiators) such as sodium persulfate, and α- and/or β-aryl nitrogen compounds (slow co-initiators) such as phenyl derivatives of glycine. Cell-adhesion ligands and cell-degradable cross-linking peptides may be incorporated into a PEG hydrogels of variable thickness, including hydrogels covalently coupled to a polystyrene-based biomedical device substrates. Hydrogels confer specific and chemically defined cell adhesion properties, as well as physiologically desirable physical properties (for example, mechanical stiffness and degradability) to otherwise standard biomedical devices. Photoinitiation and cross-linking may be performed in aqueous conditions and under ambient light, thereby adding to the value of the presently disclosed technology. Hydrogel coatings and pre-packaging of cell culture substrates of the present disclosure provide improvements in primary cell culture that are challenging to accomplish using conventional cell culture materials. Hydrogel coatings of the present disclosure may be used with micro-carriers for expansion of adherent cell types in suspension bioreactor systems. In some embodiments, the present disclosure provides encapsulation of cells within hydrogels *via* visible light cross-linking with substantial advantages over other cross-linking approaches (for example, conventional chemically initiated or UV-initiated cross-linking approaches).

US 2015/291929 A1 discloses hydrogel Compositions and methods of using hydrogel compositions. Chien-Chi Lin et al., Journal of Applied Polymer Science, vol. 132, no.8, pages 41563-1 discloses thiol-norbornene photoclick hydrogels as a diverse material system for tissue engineering applications, wherein these hydrogels are crosslinked through light-mediated orthogonal reactions between multifunctional norbornene modified macromers (e.g. poly(ethylene glocyol) (PEG), hyaluronic acid, gelatin) and sulfuhdryl-containing linkers (e.g. dithiothreitol, PEG-dithiol, bicysteine peptides) with a low concentration of photoinitiator.

### SUMMARY OF THE DISCLOSURE

The invention is defined in the appended claims. The present disclosure provides a composition, comprising a visible light harvesting complex, a photoinitiator, a co-initiator, a di-thiol-terminated cross-linker, and at least one cysteine-containing peptide. The visible light harvesting complex is RuBPY3. The photoinitiator is sodium persulfate (SPS). The co-initiator is an aryl amine. In further embodiments, the aryl amine is N-phenyl glycine. In preferred embodiments, the composition comprises 0.03 mg/mL to 3.0 mg/mL RuBPY3, 0.05 mg/mL to 5.0 mg/mL SPS, and 0.1 mg/mL to 10.0 mg/mL N-phenyl glycine. In additional embodiments, the composition further comprises at least one cell selected from the group consisting of a human umbilical vein endothelial cell, a human induced pluripotent stem cell (iPS)-derived endothelial cell, a human iPS-derived retinal pigment epithelial cell, a human iPS-derived neuron, a human iPS-derived mesenchymal stem cell, a human iPS-derived photoreceptor cell, a human iPS-derived neural stem cell, a human iPS-derived microglia, and a human iPS-derived pericyte.

Further disclosed is a composition, comprising a substrate having a surface, and a film of a cross-linked PEG endothelial tubulogenesis promoting hydrogel coating the surface of the substrate, comprising PEG-NB, and a degradable cross-linker. In certain embodiments, the composition comprises 40 - 60 mg/mL PEG-NB, 4mM - 12mM degradable KCGGPQGIWGQGCK cross-linker, 0.1 mM - 2.0 mM cyclic RGD, and 0.35 and 4.0 mM linear RGD. In other embodiments, the film of a cross-linked PEG comprises 30% to 70% by weight PEG.

Further disclosed is a method of generating a visible light photopolymerized hydrogel, comprising providing a composition, comprising a visible light harvesting complex, a photoinitiator, a co-initiator, a di-thiol-terminated cross-linker, and at least one cysteine-containing peptide, and providing a multi-armed poly(ethylene glycol) norborene, providing a surface, and photopolymerizing the multi-armed poly(ethylene glycol) norborene with visible light to form the visible light phtopolymerized hydrogel wherein the visible light harvesting complex is attached to the surface. In certain embodiments, the visible light harvesting complex is attached to the surface by a thiolated substrate surface and a norborene-functionalized poly(ethylene glycol) polymer chain. In other embodiments, the visible light harvesting complex is attached to the surface by a phenol-containing substrate surface and a norborene-functionalized poly(ethylene glycol) polymer chain. In further embodiments that method comprises generating a 3-dimensional endothelial network comprising the visible light phtopolymerized hydrogel. In additional embodiments the method comprises generating a hydrogel network comprising the visible light photopolymerized hydrogel comprising at least one cell selected from the group consisting of a human umbilical vein endothelial cell, a human iPS-derived endothelial cell, a human iPS-derived retinal pigment epithelial cell, a human iPS-derived neuron, a human iPS-derived mesenchymal stem cell, a human iPS-derived photoreceptor cell, a human iPS-derived neural stem cell, a human iPS-derived microglia, and a human iPS-derived pericyte.

### BRIEF DESCRIPTION OF THE FIGURES

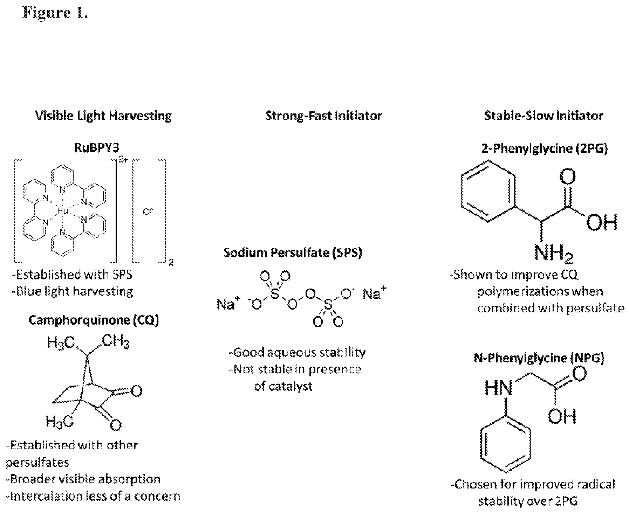

Figure 1 shows the terms, structures, classifications and rational for visible light photoinitiation reagent selection.

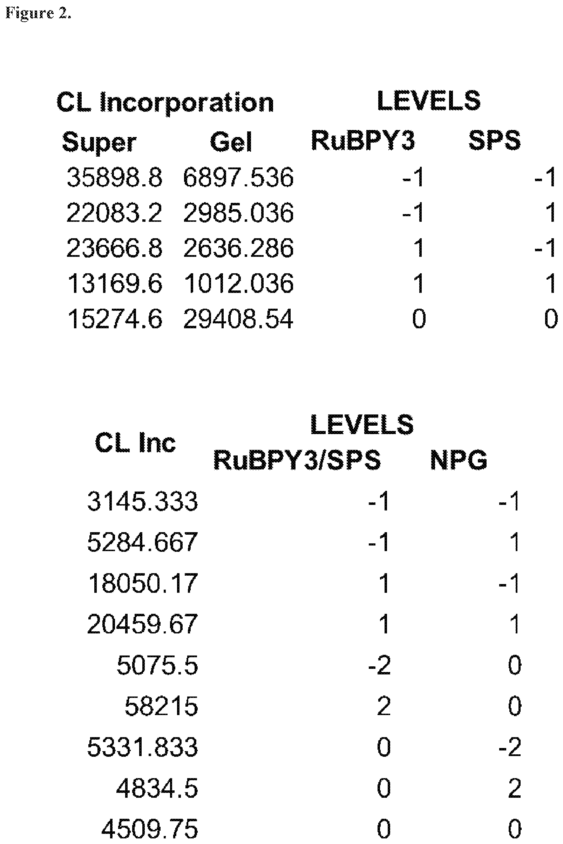

Figure 2 shows OPA detection of supernatant and incorporated peptide CL for visible light. Red corresponds to high CL detected and high concentration level of reagent.

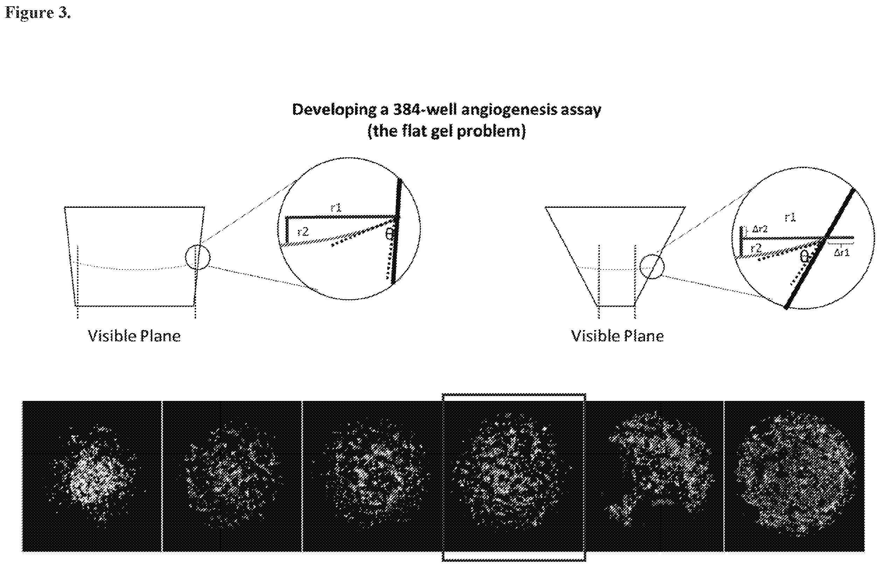

Figure 3 (right top) shows a schematic diagram that depicts competing interactions between interfaces that contribute to meniscus formation. Figure 3 (bottom) shows a schematic diagram that depicts changes in wall geometry that influence meniscus shape. Resulting flatness of 6, 5, 4, 3, 2, and 1µL UV hydrogel formulations are shown in a Greiner LoBase 384 well plate (that is, a low wall steepness plate). The red box indicates the volume that reproducibly covered the well bottom and resulted in flat hydrogels.

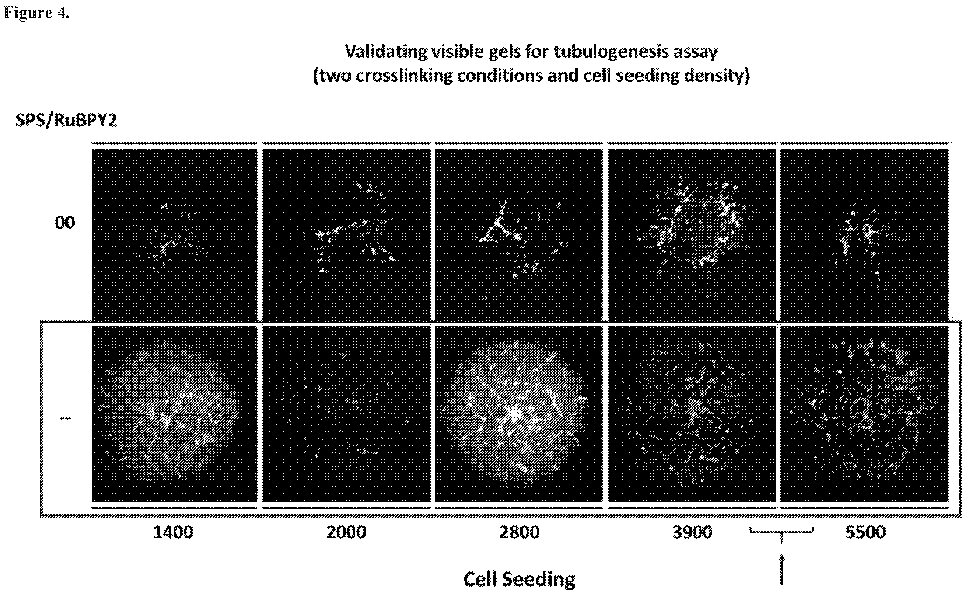

Figure 4 shows a visible light gel tubulogenesis assay. The center point and the low point conditions for SPS and RuBPY₃ were tested with varied cell density. The low center point condition had superior performance and was selected. An optimum cell density between 3900 and 5500 (for example, 4500 cells per 384 well) was selected for future studies.

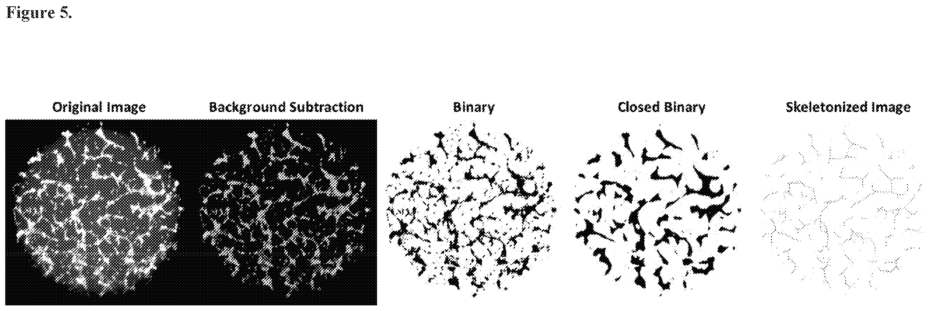

Figure 5 shows a FIJI automated tubulogenesis schematic. The steps were recorded into a macro, and batch-executed on IC50 experiment files.

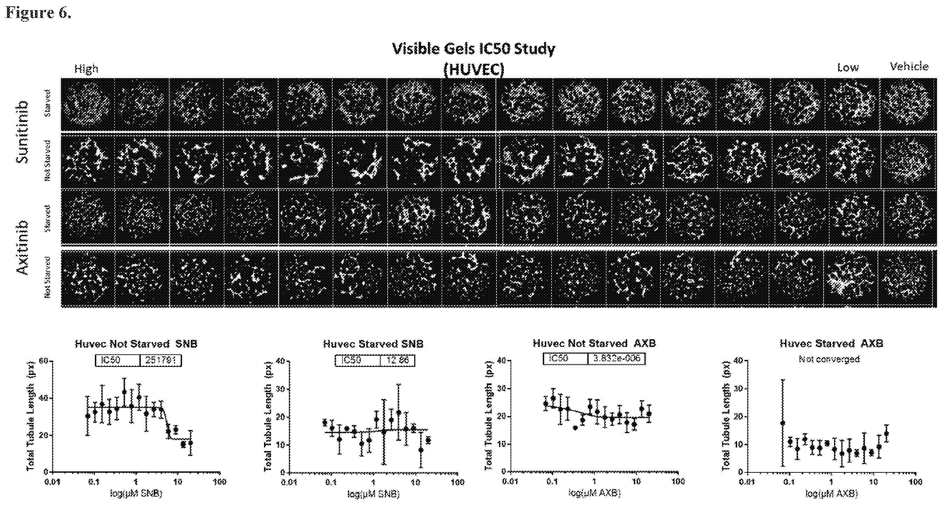

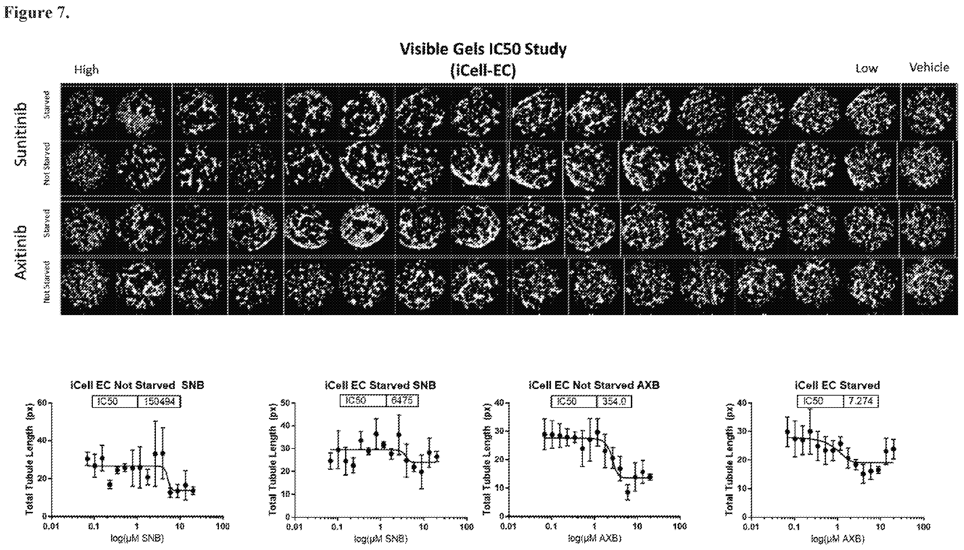

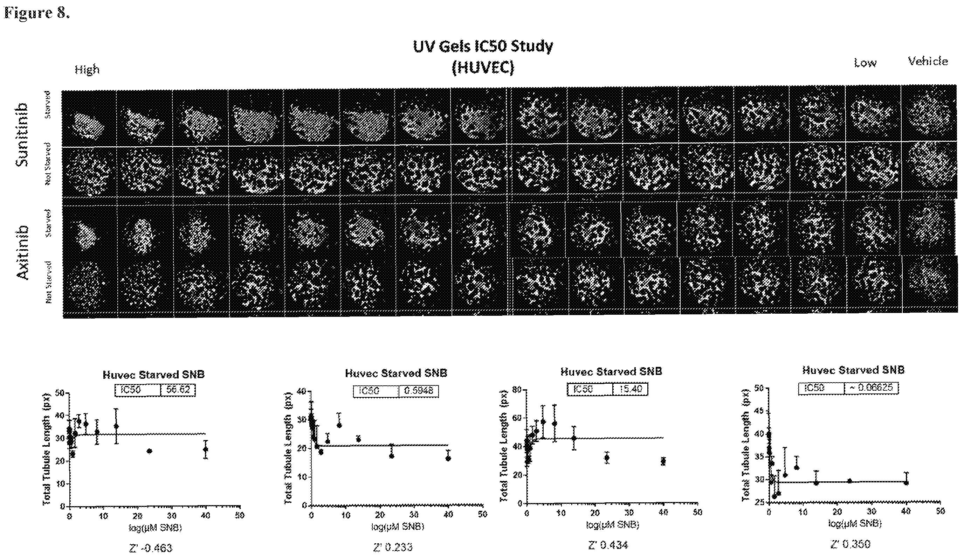

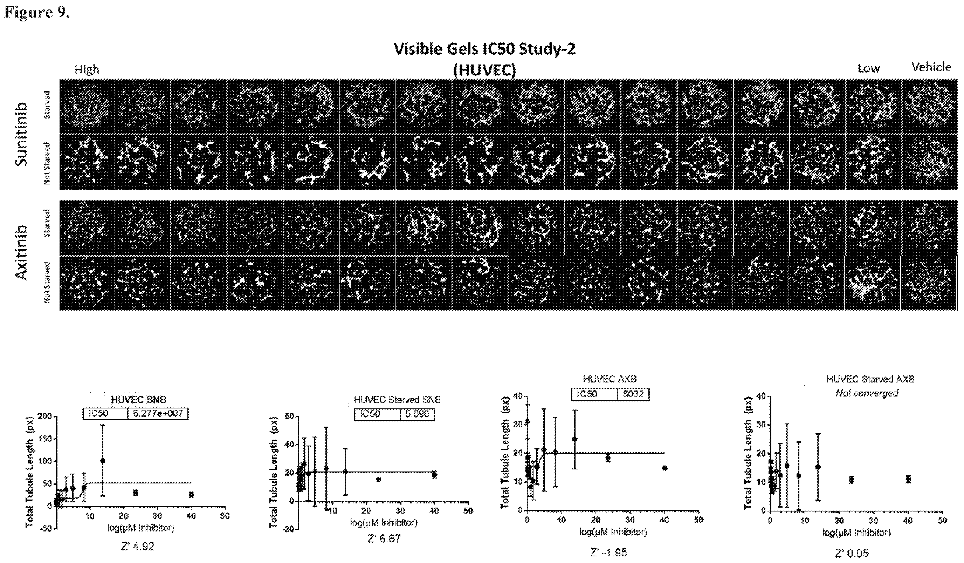

Figures 6-9 show IC50 images, resulting curves, and Z' scores for human vascular endothelial cells (HUVEC) and iCell-EC tubulogenesis in the presence of Axitinib and Sunitinib on UV and visible light-photo initiated gels.

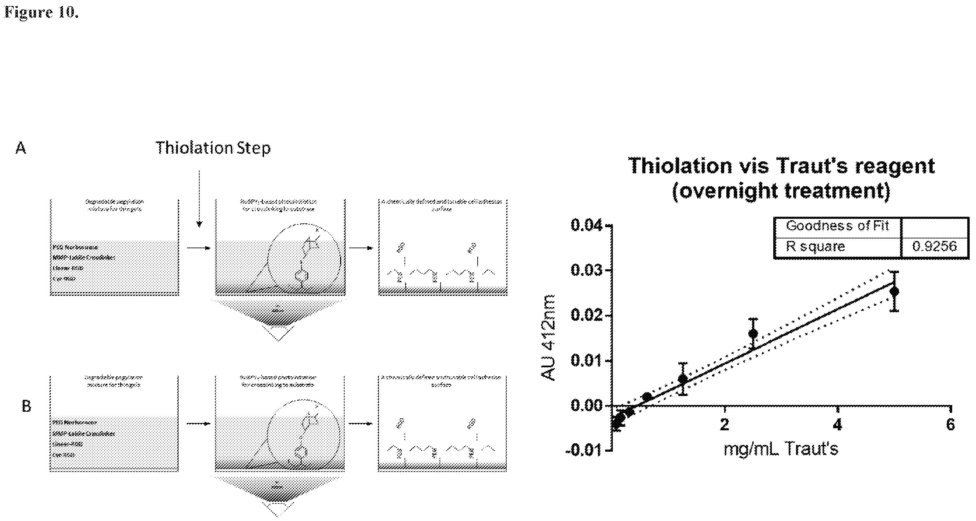

Figure 10 (left) shows schematic diagrams of two strategies for conjugating thin hydrogels onto tissue culture-treated polystyrene. Figure 10 (right) shows Ellman's assay on overnight Traut's reagent treatment of tissue culture-treated polystyrene.

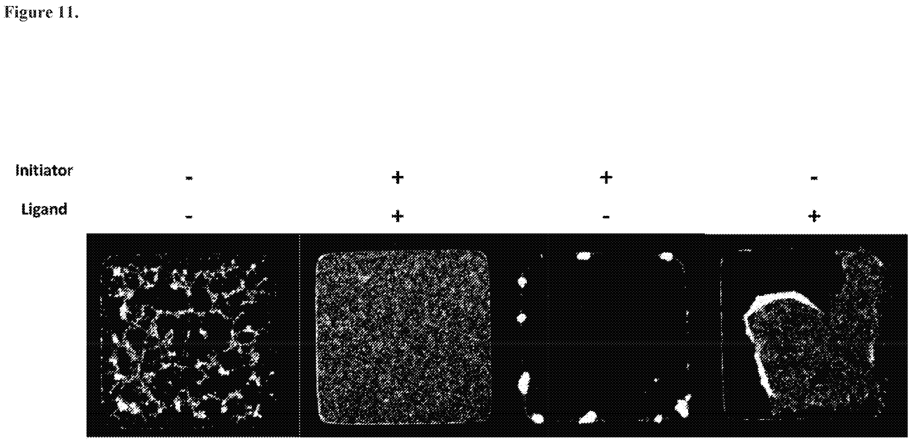

Figure 11 shows control experiments with evidence of gel cross-linking directly to the surface. Untreated tissue culture polystyrene was incubated with visible light photoinitiated hydrogel precursor solutions lacking a reagent as described. The wells were then exposed to white visible light (lamp) for 10min and then incubated for 1 hr. in PBS. CellMASK-labeled HUVECs were seeded at 5000 cells per well and then imaged after 24hrs. "Ligand" denotes the presence of cysteine terminated RGD peptide for cell attachment. "Initiator" denotes the presence of the sodium persulfate photoinitiator.

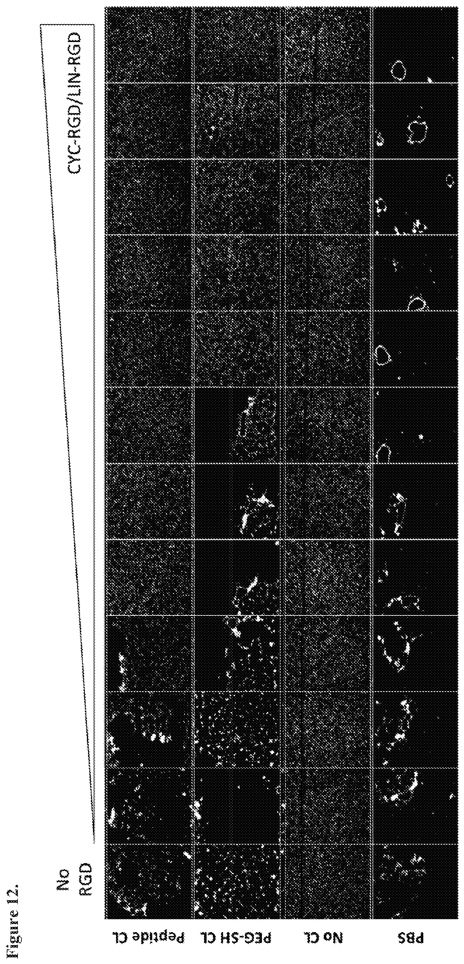

Figure 12 shows RGD ligands with different cross-linkers for thin hydrogels. The red box indicates a concentration region tubule formation that was selected for further experimentation.

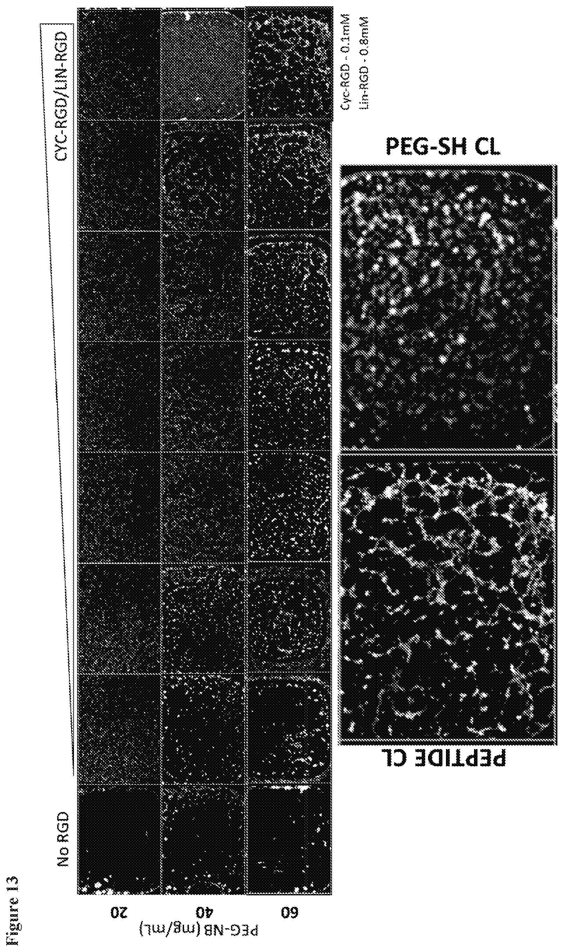

Figure 13 shows titration of RGD ligands with peptide cross-linkers at different ratios to PEG-NB. The highest concentration of RGD for 60mg/mL PEG-NB to 4mM thiol concentration from cross-linker resulted in tubules with preferred morphology. (bottom) The equivalent concentration of RGD ligands with non-degradable cross-linkers resulted in monolayer formation. The red box indicates the selected condition of 0.1mM cyc-RGD, 0.8mM lin-RGD, 60mg/mL PEG-NB, and 4mM thiol from the peptide cross-linker.

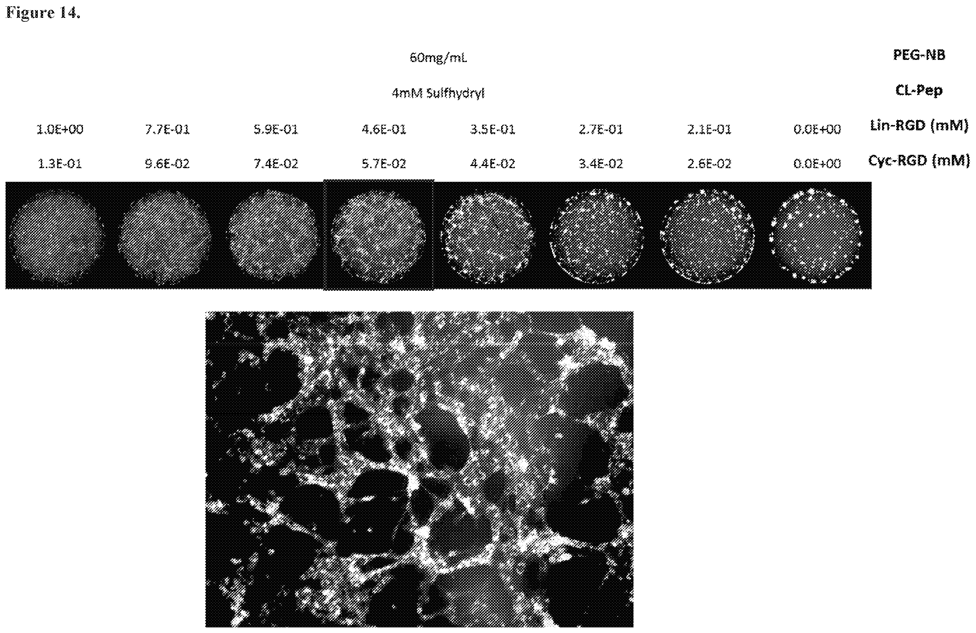

Figure 14 shows titration of RGD ligands with peptide cross-linker in the Greiner LoBase plate (smaller area). The concentration of RGD in the formulation was further reduced, and tubules identified at the condition indicated by the red box. (bottom) A 10X view of thin gel tubule formation from the indicated red box is provided.

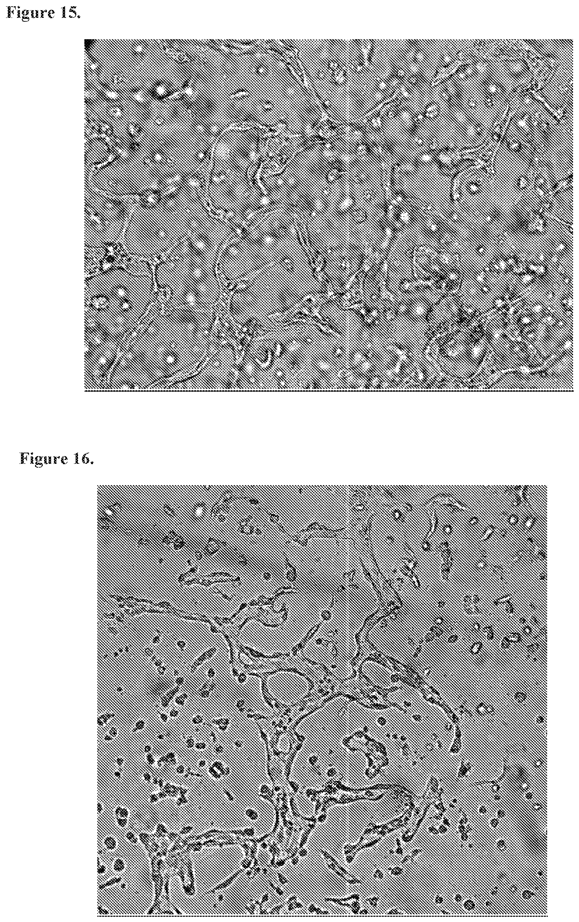

Figure 15 shows iCell Endothelial cells embedded in a hydrogel of the present disclosure using visible light cross-linking methods. The image was taken 7 days after embedding the cells. Stable 3D vascular networks formed in the hydrogel.
Figure 16 shows HUVEC cells plated onto a hydrogel formulation that promotes tubulogenesis. At 24 hours, the cell medium was removed, and a hydrogel overlay was formed on top of the cells. The hydrogel overlay maintained the tubule structures for 6 days. The image was taken 7 days after plating the cells.

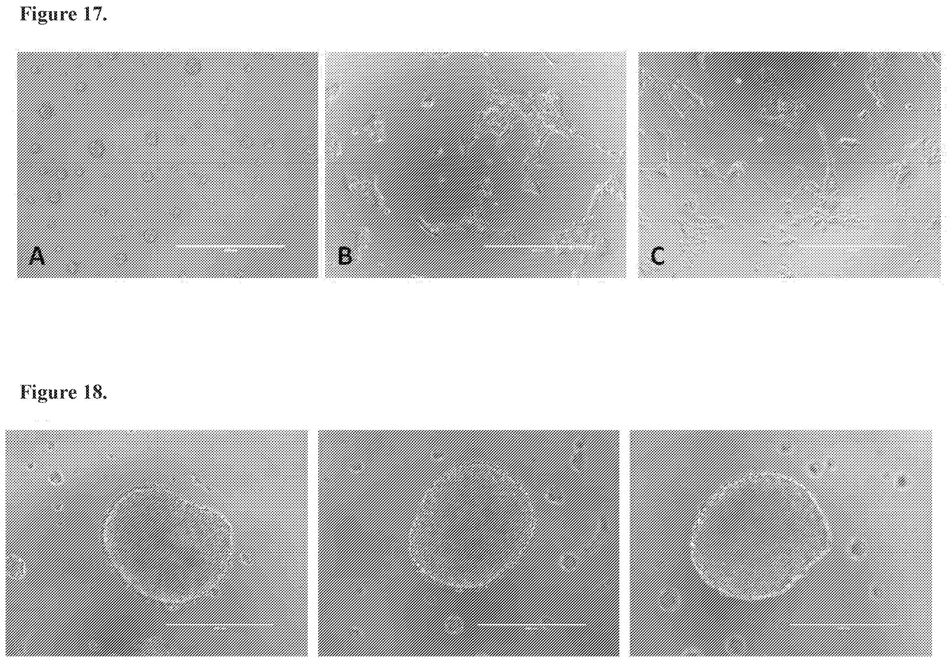

Figure 17 shows, for reference only and not according to the claimed invention, human neural stem cells derived from h9 ES cells that were plated onto tissue culture plastic (panel A), which does not support NSC adhesion; onto a thin coating of Geltrex, EHS-derived matric, diluted 1:100 and absorbed to the bottom of a 96 well plate, which supports NSC adhesion and expansion (panel B); or onto a thin hydrogel which supports NSC adhesion and expansion (panel C). The images were taken two days after plating the cells.
Figure 18 shows, for reference only and not according to the claimed invention, examples of three aggregates of human neural stem cells derived from h9 ES cells that are attached to the surface of a visible-light-initiated thick hydrogel formulation which supports the maintenance of that 3-dimensional organoid in a 96 wp format. The aggregates, or organoids, can be maintained and assayed in said 96 wp format. Aggregates formed are consistent in size (scale bar shown represents 400 um).

### DEFINITIONS

As used herein, the term "cell" refers to any eukaryotic or prokaryotic cell (*e.g.*, bacterial cells such as *E. coli,* yeast cells, mammalian cells, avian cells, amphibian cells, plant cells, fish cells, and insect cells), whether located *in vitro* or *in vivo.* For example, cells may be located in a transgenic animal.

As used herein, the term "subject" refers to individuals (*e.g.*, human, animal, or other organism) to be treated by the methods or compositions of the present disclosure. Subjects include, but are not limited to, mammals (*e.g.*, murines, simians, equines, bovines, porcines, canines, felines, and the like), and most preferably includes humans.

As used herein the term, "*in vitro*" refers to an artificial environment and to processes or reactions that occur within an artificial environment. *In vitro* environments include, but are not limited to, test tubes and cell cultures. The term "*in vivo*" refers to the natural environment (*e.g.*, an animal or a cell) and to processes or reaction that occur within a natural environment.

As used herein, the term "effective amount" refers to the amount of a composition sufficient to effect beneficial or desired results. An effective amount can be administered in one or more administrations, applications or dosages and is not intended to be limited to a particular formulation or administration route.

As used herein, the term "administration" refers to the act of giving a drug, prodrug, or other agent, or therapeutic treatment to a physiological system (*e.g.*, a subject or *in vivo, in vitro,* or *ex vivo* cells, tissues, and organs). Exemplary routes of administration to the human body can be through the eyes (ophthalmic), mouth (oral), skin (transdermal), nose (nasal), lungs (inhalant), oral mucosa (buccal), ear, by injection (*e.g.*, intravenously, subcutaneously, intratumorally, intraperitoneally, *etc*.), topical administration and the like.

As used herein, the term "co-administration" refers to the administration of at least two agent(s) or therapies to a subject. In some embodiments, the co-administration of two or more agents or therapies is concurrent. In other embodiments, a first agent/therapy is administered prior to a second agent/therapy. Those of skill in the art understand that the formulations and/or routes of administration of the various agents or therapies used may vary. The appropriate dosage for co-administration can be readily determined by one skilled in the art. In some embodiments, when agents or therapies are co-administered, the respective agents or therapies are administered at lower dosages than appropriate for their administration alone. Thus, co-administration is especially desirable in embodiments where the co-administration of the agents or therapies lowers the requisite dosage of a potentially harmful (*e.g.*, toxic) agent(s).

As used herein, the term "pharmaceutical composition" refers to the combination of an active agent (*e.g.*, bacteria described herein) with a carrier, inert or active, making the composition especially suitable for diagnostic or therapeutic use *in vitro, in vivo* or *ex vivo.*

The terms "pharmaceutically acceptable" or "pharmacologically acceptable," as used herein, refer to compositions that do not substantially produce adverse reactions, e.g., toxic, allergic, or immunological reactions, when administered to a subject.

As used herein, the term "pharmaceutically acceptable carrier" refers to any of the standard pharmaceutical carriers including, but not limited to, phosphate buffered saline solution, water, emulsions (*e.g.*, such as an oil/water or water/oil emulsions), and various types of wetting agents, any and all solvents, dispersion media, coatings, sodium lauryl sulfate, isotonic and absorption delaying agents, disintrigrants (*e.g.*, potato starch or sodium starch glycolate), and the like. The compositions also can include stabilizers and preservatives. For examples of carriers, stabilizers, and adjuvants. (See *e.g.,* Martin, Remington's Pharmaceutical Sciences, 15th Ed., Mack Publ. Co., Easton, Pa. (1975).

In certain embodiments, the compositions of the present disclosure may be formulated for veterinary, horticultural or agricultural use. Such formulations include dips. sprays, seed dressings, stem injections, sprays, and mists. In certain embodiments, compositions of the present disclosure may be used in any application where it is desirable to alter (e.g., inhibit) the formation of biofilms, e.g., food industry applications; consumer goods (e.g., medical goods, goods intended for consumers with impaired or developing immune systems (e.g., infants, children, elderly, consumers suffering from disease or at risk from disease), and the like.

As used herein, the term "therapeutic agent," refers to compositions that decrease the infectivity, morbidity, or onset of mortality in a subject (e.g., a transplant recipient) or that prevent infectivity, morbidity, or onset of mortality in a host. As used herein, therapeutic agents encompass agents used prophylactically, *e.g.*, before transplant. Such agents may additionally comprise pharmaceutically acceptable compounds (*e.g.*, adjuvants, excipients, stabilizers, diluents, and the like). In some embodiments, the therapeutic agents of the present disclosure are administered in the form of topical compositions, injectable compositions, ingestible compositions, and the like. When the route is topical, the form may be, for example, a solution, cream, ointment, salve or spray.

As used herein, the term "cell culture" refers to any *in vitro* culture of cells, including, *e.g.*, prokaryotic cells and eukaryotic cells. Included within this term are continuous cell lines (*e.g.*, with an immortal phenotype), primary cell cultures, transformed cell lines, finite cell lines (*e.g.*, non-transformed cells), bacterial cultures in or on solid or liquid media, and any other cell population maintained *in vitro.*

As used herein, the term "sample" is used in its broadest sense. In one sense, it is meant to include a specimen or culture obtained from any source, as well as biological and environmental samples. Biological samples may be obtained from animals (including humans) and encompass fluids, solids, tissues, and gases. Biological samples include blood products, such as plasma, serum and the like. Such examples are not however to be construed as limiting the sample types applicable to the present disclosure.

In the specification and in the claims, the terms "including" and "comprising" are openended terms and should be interpreted to mean "including, but not limited to.... " These terms encompass the more restrictive terms "consisting essentially of" and "consisting of."

It must be noted that as used herein and in the appended claims, the singular forms "a", "an", and "the" include plural reference unless the context clearly dictates otherwise. As well, the terms "a" (or "an"), "one or more" and "at least one" can be used interchangeably herein. It is also to be noted that the terms "comprising", "including", "characterized by" and "having" can be used interchangeably.

"Alkyl" by itself or as part of another substituent, refers to a saturated or unsaturated, branched, straight-chain or cyclic monovalent hydrocarbon radical derived by the removal of one hydrogen atom from a single carbon atom of a parent alkane, alkene or alkyne. Typical alkyl groups include, but are not limited to, methyl; ethyls such as ethanyl, ethenyl, ethynyl; propyls such as propan-1-yl, propan-2-yl, cyclopropan-1-yl, prop-1-en-1-yl, prop-1-en-2-yl, prop-2-en-1-yl (allyl), cycloprop-1-en-1-yl; cycloprop-2-en-1-yl, prop-1-yn-1-yl , prop-2-yn-1-yl, *etc.;* butyls such as butan-1-yl, butan-2-yl, 2-methyl-propan-1-yl, 2-methyl-propan-2-yl, cyclobutan-1-yl, but-1-en-1-yl, but-1-en-2-yl, 2-methyl-prop-1-en-1-yl, but-2-en-1-yl , but-2-en-2-yl, buta-1,3-dien-1-yl, buta-1,3-dien-2-yl, cyclobut-1-en-1-yl, cyclobut-1-en-3-yl, cyclobuta-1,3-dien-1-yl, but-1-yn-1-yl, but-1-yn-3-yl, but-3-yn-1-yl, *etc.;* and the like.

The term "alkyl" is specifically intended to include groups having any degree or level of saturation, *i.e.*, groups having exclusively single carbon-carbon bonds, groups having one or more double carbon-carbon bonds, groups having one or more triple carbon-carbon bonds and groups having mixtures of single, double and triple carbon-carbon bonds. Where a specific level of saturation is intended, the expressions "alkanyl," "alkenyl," and "alkynyl" are used. Preferably, an alkyl group comprises from 1 to 15 carbon atoms (C₁-C₁₅ alkyl), more preferably from 1 to 10 carbon atoms (C₁-C₁₀ alkyl) and even more preferably from 1 to 6 carbon atoms (C₁-C₆ alkyl or lower alkyl).

"Alkanyl," by itself or as part of another substituent, refers to a saturated branched, straight-chain or cyclic alkyl radical derived by the removal of one hydrogen atom from a single carbon atom of a parent alkane. Typical alkanyl groups include, but are not limited to, methanyl; ethanyl; propanyls such as propan-1-yl, propan-2-yl (isopropyl), cyclopropan-1-yl, etc.; butanyls such as butan-1-yl, butan-2-yl (*sec*-butyl), 2-methyl-propan-1-yl (isobutyl), 2-methyl-propan-2-yl (*t*-butyl), cyclobutan-1-yl, *etc.;* and the like.

"Alkenyl," by itself or as part of another substituent, refers to an unsaturated branched, straight-chain or cyclic alkyl radical having at least one carbon-carbon double bond derived by the removal of one hydrogen atom from a single carbon atom of a parent alkene. The group may be in either the *cis* or *trans* conformation about the double bond(s). Typical alkenyl groups include, but are not limited to, ethenyl; propenyls such as prop-1-en-1-yl , prop-1-en-2-yl, prop-2-en-1-yl (allyl), prop-2-en-2-yl, cycloprop-1-en-1-yl; cycloprop-2-en-1-yl ; butenyls such as but-1-en-1-yl, but-1-en-2-yl, 2-methyl-prop-1-en-1-yl, but-2-en-1-yl , but-2-en-1-yl, but-2-en-2-yl, buta-1,3-dien-1-yl, buta-1,3-dien-2-yl, cyclobut-1-en-1-yl, cyclobut-1-en-3-yl, cyclobuta-1,3-dien-1-yl, *etc.;* and the like.

"Alkyldiyl" by itself or as part of another substituent refers to a saturated or unsaturated, branched, straight-chain or cyclic divalent hydrocarbon group derived by the removal of one hydrogen atom from each of two different carbon atoms of a parent alkane, alkene or alkyne, or by the removal of two hydrogen atoms from a single carbon atom of a parent alkane, alkene or alkyne. The two monovalent radical centers or each valency of the divalent radical center can form bonds with the same or different atoms. Typical alkyldiyl groups include, but are not limited to, methandiyl; ethyldiyls such as ethan-1,1-diyl, ethan-1,2-diyl, ethen-1,1-diyl, ethen-1,2-diyl; propyldiyls such as propan-1,1-diyl, propan-1,2-diyl, propan-2,2-diyl, propan-1,3-diyl, cyclopropan-1,1-diyl, cyclopropan-1,2-diyl, prop-1-en-1,1-diyl, prop-1-en-1,2-diyl, prop-2-en-1,2-diyl, prop-1-en-1,3-diyl, cycloprop-1-en-1,2-diyl, cycloprop-2-en-1,2-diyl, cycloprop-2-en-1,1-diyl, prop-1-yn-1,3-diyl, etc.; butyldiyls such as, butan-1,1-diyl, butan-1,2-diyl, butan-1,3-diyl, butan-1,4-diyl, butan-2,2-diyl, 2-methyl-propan-1,1-diyl, 2-methyl-propan-1,2-diyl, cyclobutan-1,1-diyl; cyclobutan-1,2-diyl, cyclobutan-1,3-diyl, but-1-en-1,1-diyl, but-1-en-1,2-diyl, but-1-en-1,3-diyl, but-1-en-1,4-diyl, 2-methyl-prop-1-en-1,1-diyl, 2-methanylidene-propan-1,1-diyl, buta-1,3-dien-1,1-diyl, buta-1,3-dien-1,2-diyl, buta-1,3-dien-1,3-diyl, buta-1,3-dien-1,4-diyl, cyclobut-1-en-1,2-diyl, cyclobut-1-en-1,3-diyl, cyclobut-2-en-1,2-diyl, cyclobuta-1,3-dien-1,2-diyl, cyclobuta-1,3-dien-1,3-diyl, but-1-yn-1,3-diyl, but-1-yn-1,4-diyl, buta-1,3-diyn-1,4-diyl, etc.; and the like. Where specific levels of saturation are intended, the nomenclature alkanyldiyl, alkenyldiyl and/or alkynyldiyl is used. Where it is specifically intended that the two valencies are on the same carbon atom, the nomenclature "alkylidene" is used. In preferred embodiments, the alkyldiyl group comprises from 1 to 6 carbon atoms (C1-C6 alkyldiyl). Also preferred are saturated acyclic alkanyldiyl groups in which the radical centers are at the terminal carbons, *e.g.,* methandiyl (methano); ethan-1,2-diyl (ethano); propan-1,3-diyl (propano); butan-1,4-diyl (butano); and the like (also referred to as alkylenos, defined *infra*).

"Alkyleno," by itself or as part of another substituent, refers to a straight-chain saturated or unsaturated alkyldiyl group having two terminal monovalent radical centers derived by the removal of one hydrogen atom from each of the two terminal carbon atoms of straight-chain parent alkane, alkene or alkyne. The locant of a double bond or triple bond, if present, in a particular alkyleno is indicated in square brackets. Typical alkyleno groups include, but are not limited to, methano; ethylenos such as ethano, etheno, ethyno; propylenos such as propano, prop[1]eno, propa[1,2]dieno, prop[1]yno, etc.; butylenos such as butano, but[1]eno, but[2]eno, buta[1,3]dieno, but[1]yno, but[2]yno, buta[1,3]diyno, etc.; and the like. Where specific levels of saturation are intended, the nomenclature alkano, alkeno and/or alkyno is used. In preferred embodiments, the alkyleno group is (C1-C6) or (C1-C3) alkyleno. Also preferred are straight-chain saturated alkano groups, e.g., methano, ethano, propano, butano, and the like.

"Alkylene" by itself or as part of another substituent refers to a straight-chain saturated or unsaturated alkyldiyl group having two terminal monovalent radical centers derived by the removal of one hydrogen atom from each of the two terminal carbon atoms of straight-chain parent alkane, alkene or alkyne. The locant of a double bond or triple bond, if present, in a particular alkylene is indicated in square brackets. Typical alkylene groups include, but are not limited to, methylene (methano); ethylenes such as ethano, etheno, ethyno; propylenes such as propano, prop[1]eno, propa[1,2]dieno, prop[1]yno, etc.; butylenes such as butano, but[1]eno, but[2]eno, buta[1,3]dieno, but[1]yno, but[2]yno, buta[1,3]diyno, etc.; and the like. Where specific levels of saturation are intended, the nomenclature alkano, alkeno and/or alkyno is used. In preferred embodiments, the alkylene group is (C1-C6) or (C1-C3) alkylene. Also preferred are straight-chain saturated alkano groups, e.g., methano, ethano, propano, butano, and the like.

"Substituted," when used to modify a specified group or radical, means that one or more hydrogen atoms of the specified group or radical are each, independently of one another, replaced with the same or different substituent(s). Substituent groups useful for substituting saturated carbon atoms in the specified group or radical include, but are not limited to -R^{a}, halo, -O⁻, =O, -OR^{b}, -SR^{b}, -S⁻, =S, -NR^{c}R^{c}, =NR^{b}, =N-OR^{b}, trihalomethyl, -CF₃, -CN, -OCN, -SCN, -NO, -NO₂,=N₂, -N₃, -S(O)₂R^{b}, -S(O)₂O⁻, -S(O)₂OR^{b}, -O S(O)₂R^{d}, -OS(O)₂O⁻, -OS(O)₂OR^{b}, -P(O)(O⁻)₂, -P(O)(OR^{b})(O⁻), -P(O)(OR^{b})(OR^{b}), -C(O)R^{b}, -C(S )R^{b}, -C(NR^{b})R^{b}, -C(O)O⁻, -C(O)OR^{b}, -C(S)OR^{b}, -C(O)NR^{c}R^{c}, -C(NR^{b})NR^{c}R^{c}, -OC(O)R^{b}, -OC(S )R^{b}, -OC(O)O⁻, -OC(O)OR^{b}, -OC(S)OR^{b}, -NR^{b}C(O)R^{b}, -NR^{b}C(S)R^{b}, -NR^{b}C(O)O⁻, -NR^{b}C(O)OR ^{b}, -NR^{b}C(S)OR^{b}, -NR^{b}C(O)NR^{c}R^{c}, -NR^{b}C(NR^{b})R^{b} and -NR^{b}C(NR^{b})NR^{c}R^{c}, where R^{a} is selected from the group consisting of alkyl, cycloalkyl, heteroalkyl, cycloheteroalkyl, aryl, arylalkyl, heteroaryl and heteroarylalkyl; each R^{d} is independently hydrogen or R^{a}; and each R^{c} is independently R^{b} or alternatively, the two R^{c}s are taken together with the nitrogen atom to which they are bonded form a 5-, 6- or 7-membered cycloheteroalkyl which may optionally include from 1 to 4 of the same or different additional heteroatoms selected from the group consisting of O, N and S. As specific examples, -NR^{c}R^{c} is meant to include -NH₂, -NH-alkyl, N-pyrrolidinyl and N-morpholinyl.

Similarly, substituent groups useful for substituting unsaturated carbon atoms in the specified group or radical include, but are not limited to, -R^{a}, halo, -O⁻, -OR^{b}, -SR^{b}, -S⁻, -NR^{c}R^{c}, trihalomethyl, -CF₃, -CN, -OCN, -SCN, -NO, -NO₂, -N₃, -S(O)₂R^{b}, -S(O)₂O⁻, -S(O)₂OR^{b}, -OS(O) ₂R^{b}, -OS(O)₂O⁻, -OS(O)₂OR^{d}, -P(O)(O⁻)₂, -P(O)(OR^{b})(O⁻), -P(O)(OR^{b})(OR^{b}), -C(O)R^{b}, -C(S)R^{b}, -C(NR^{b})R^{b}, -C(O)O⁻, -C(O)OR^{b}, -C(S)OR^{b}, -C(O)NR^{c}R^{c}, -C(NR^{b})NR^{c}R^{c}, -OC(O)R^{b}, -OC(S)R^{b}, -OC(O)O⁻, -OC(O)OR^{b}, -OC(S)OR^{b}, -NR^{b}C(O)R^{b}, -NR^{b}C(S)R^{b}, -NR^{b}C(O)O⁻, -NR^{b}C(O)OR^{b},-NR^{b}C(S)OR^{b}, -NR^{b}C(O)NR^{c}R^{c}, -NR^{b}C(NR^{b})R^{b} and -NR^{b}C(NR^{b})NR^{c}R^{c}, where R^{a}, R^{b} and R^{c} are as previously defined.

Substituent groups useful for substituting nitrogen atoms in heteroalkyl and cycloheteroalkyl groups include, but are not limited to, -R^{a}, -O⁻, -OR^{b}, -SR^{b}, -S⁻, -NR^{c}R^{c}, trihalomethyl, -CF₃, -CN, -NO, -NO₂, -S(O)₂R^{b}, -S(O)₂O⁻, -S(O)₂OR^{b}, -OS(O)₂R^{b}, -OS(O)₂O⁻,-OS(O)₂OR^{d}, -P(O)(O⁻)₂, -P(O)(OR^{b})(O⁻), -P(O)(OR^{b})(OR^{b}), -C(O)R^{b}, -C(S)R^{b}, -C(NR^{b})R^{b}, -C(O )OR^{b}, -C(S)OR^{b}, -C(O)NR^{c}R^{c}, -C(NR^{b})NR^{c}R^{c}, -OC(O)R^{b}, -OC(S)R^{b}, -OC(O)OR^{b}, -OC(S)OR^{b},-NR^{b}C(O)R^{b}, -NR^{b}C(S)R^{b}, -NR^{b}C(O)OR^{b}, -NR^{b}C(S)OR^{b}, -NR^{b}C(O)NR^{c}R^{c}, -NR^{b}C(NR^{b})R^{b} and -NR^{b}C(NR^{b})NR^{c}R^{c}, where R^{a}, R^{b} and R^{c} are as previously defined.

Substituent groups from the above lists useful for substituting other specified groups or atoms will be apparent to those of skill in the art.

The substituents used to substitute a specified group can be further substituted, typically with one or more of the same or different groups selected from the various groups specified above.

By "3-dimensional," is meant that the hydrogel is not substantially flat (*i.e.,* not 2-dimensional) such that cells associated with the hydrogel may be in contact with hydrogel surfaces both above and below the cells.

As used herein, a "thin gel" is any gel thickness less than or equal to 60 um in depth. A "thick gel" is any gel thickness greater than 60 um in depth. As demonstrated by Merkel *et al.* this is the thickness wherein finite thickness effects are relevant (Biophysical Journal, Vol. 93 November (2007); 3314-3323). In some embodiments, thick gels are 200 um to 2 mm in thickness. Gel thickness may be measured by physical measurement (*e.g.*, by microcaliper), or by optical techniques (*e.g.,* a combination of fluorescent dyes and/or fluorescent microscopy, confocal microscopy, or atomic force microscopy), or by other suitable technology. Processes for generating thick gels *vs.* thin gels are distinguished in that a thick gel is, in certain embodiments, a free-standing gel with a structure derived from the cross-linking of polymers. In other embodiments, a thin gel is formed by a reaction at the surface of a substrate, either through a chemical interaction with the surface, or through a localized photoinitiation event, and/or by limiting a cross-linker concentration to prevent the formation of a thick gel initiating from the surface.

Unless defined otherwise, all technical and scientific terms used herein have the same meanings as commonly understood by one of ordinary skill in the art to which this invention belongs. All references cited in this specification are to be taken as indicative of the level of skill in the art. Nothing herein is to be construed as an admission that the invention is not entitled to antedate such disclosure by virtue of prior invention.

### DETAILED DESCRIPTION

There is widespread interest in forming hydrogels for biomedical applications, including use as customized cell culture substrates, matrices for *ex vivo* tissue assembly, injectable carriers for cell therapy, and as implantable carriers for cell or tissue therapy. In particular biomedical applications there is a need for hydrogels that may be cross-linked by visible light. The present disclosure provides chemistries for cross-linking poly(ethylene glycol) hydrogels using visible light, as defined in the appended claims. The chemistries combines a visible light harvesting complex, a photoinitiator, a co-initiator, a di-thiol-terminated cross-linker, and one or more cysteine-containing peptides, and generate visible light photopolymerization of multi-armed poly(ethylene glycol) norborene to form hydrogels. The process may be adapted to form free-standing hydrogels of diverse thicknesses and dimensionalities, as well as thin hydrogel films formed on the surface of, for example, biomedical devices.

In some embodiments, the present invention provides a thin gel. In other embodiments, the present invention provides a thick gel. The uses and methods of making thin and thick gels differ in certain embodiments. In particular embodiments, a thick gel provides a mechanically "tunable" and flexible substrate of use, for example, for embedding cells, for generating conditions for tissue or organoid assembly, for cell expansion and differentiation, and/or for assay development. In specific embodiments, thick gels simulate a spectrum of tissue mechanical properties. In further embodiments, thin gels (for example, thin gels at the thinest end of their thickness range), acquire the mechanical properties of the surface they coat, and also provide surface chemistries that promote, for example, cell adhesion and formation of cellular structures. In still further embodiments, then gels are used for cell expansion, for assay development, for creating conditions for tissue and organoid assembly, and/or for measurement of voltage changes or membrane potentials analyzed using multi-electrode array instrumentation. In other embodiments, thick gels are polymerized by an end-user from a monomeric solution. In additional embodiments, thin gels are pre-formed at the surface of a tissue culture plate or chamber, freeze-dried and delivered to an end-user ready for re-hydration and use.

### Visible Lght Photoinitiation

A barrier to adoption of PEG-norborene (PEG-NB) hydrogels in place of Matrigel for cell-based assays (for example, tubulogenesis assays) is the capital investment required for specialized equipment including, for example, a UV lamp for photoinitiation of UV photoinitiators (for example, Irgacure I2959). Photoinitiation chemically cross-links the hydrogel *via* thiolene-based chemistry. The present disclosure provides a photoinitiation system that improves the workflow of the PEG-NB tubulogenesis assay using visible light. The use of photoinitiation is preferred over thermal and chemical photoinitiation because it provides the user with a greater degree of control. For example, the user can configure an experiment under one set of illumination conditions and, when ready, change the illumination conditions to cross-link their hydrogel. The compositions and methods of the present disclosure provide visible light photoinitiation instead of UV light photoinitiation to achieve flexibility without the need for capital investment in specialized light sources. Moreover, visible light photopolymerization is also less likely to cause toxicity to cells present within a solution during hydrogel polymerization. Visible light photoinitiation strategies are particularly advantageous in applications in which cells are encapsulated within hydrogels, including 3-dimensional cell-based assays, cell therapy, and tissue therapy.

### EXAMPLES

### Example 1 - Visible light photo-cross-linking

For visible light cross-linking, ruthenium complex blue light harvesting was selected because indoor white light sources may have significant portions of their illumination spectrum dominated by blue light, as well as the comparably much less expensive and more widespread availability of blue light emitting diode (LED) light sources over specialized UV lamps. The ruthenium based system harvests blue light and transfers energy to activate an initiator, for example, sodium persulfate (SPS). Another visible light harvesting complex, Camphorquinone (CQ), may also transfer energy to persulfate. According to the invention, the photoinitiator is SPS. SPS was combined with two aryl amine co-initiators to generate a combination fast (SPS) and slow (aryl amine) initiation system for two purposes. Slow initiators are more stable molecules particularly in the presence of the light harvesting complex, thereby improving the stability of a complete hydrogel, peptide, and photoinitiation system formulation for both working times and storage shelf life. Also, the combination generates a longer lasting radical species that improves cross-linking. To evaluate the different photoinitiation systems, each of the systems' distinct parameters were varied. Two different photoinitiation schemes were investigated. The first was ambient light in a biosafety cabinet off (dim) and on (bright). This condition may be the most desirable because it requires no capital purchase by the end user. The second condition was under blue LED light illumination. This condition requires a capital purchase by the end user, albeit at significantly less costly than a conventional UV lamp. The blue LED illumination scheme may be a specialty application in applications such as visible light encapsulation.

Based on their solubility over CQ and 2-PG, RuBPY₃, SPS, and N-phenyl glycine (N-PG) were tested as a photoinitiation system using a fluoraldehyde (OPA) (ThermoFisher) assay to detect free primary amines of the lysine side chains contained in the cross-linker as a quantitative output. OPA functioned as desired, with high cross-linker concentrations observed in the supernatant in correlation with low concentrations retained in the hydrogel (and *vice versa*). The relative molar concentration of RuBPY₃ to SPS is critical for efficient photoinitiation. (Figure 2.) Different results in surface cross-linking (for example, thin gels) as well as ambient light cross-linking with the N-PG, were observed with use of the N-PG as a "slow" co-initiator. In the presence of N-PG, lower concentrations (for example, 100 fold lower) of the RuBPY₃ and SPS complex were required for gelation of hydrogels, thin gels were thicker and induced more gel-like cellular responses, and ambient cross-linked hydrogels were less likely to degrade in response to over excitation. A lower likelihood of hydrogel degradation was observed when DMSO was included in the formulation (for example, as low as 0.5%), indicating that the over excitation/initiation either creates an oxidizing environment that reduces the stability of the ester linked norborene to the PEG backbone, or scavenges excess free radicals after monomer consumption.

### Example 2 - Tubulogenesis Assays

PEG-based formats used for tubulogenesis assays were formed *via* UV cross-linking of hydrogels in a 96 well plate. Aims for improving tubulogeneis assays included: 1) improved flatness of hydrogel surfaces for endothelial cell seeding and endothelial network formation (termed "tubulogenesis"); 2) adaptation of the assay to a 384-well plate format for a larger angiogenesis screening platform; and 3) use of visible light photoinitiation to form hydrogels and to compare tubulogenesis assay results with visible light cross-linked hydrogels to those formed *via* UV light photoinitiation. In particular, tubulogenesis experiments compared the ability of hydrogels to support tubulogenesis by human endothelial cells, and the responsiveness of the resulting endothelial networks to the VEGF inhibitors Axitinib and Sunitinib. Experiments conducted to achieve the first aim altered the hydrogel precursor solution, the walls of the plate, and the air-liquid interface interactions, and compared charged *vs.* uncharged (tissue-culture treated *vs*. untreated) well plates, use of surfactants in the precursor solution, inversion to reduce the meniscus *via* gravitational force, and use of plates with less vertical walls. Results revealed that a specific 384 well plate with a rounded geometry (LoBase plates from Greiner) met the first two performance criteria listed above. The slanted orientation of the well wall provided flat hydrogels in a 384 well format enabling use of substantially less hydrogel precursor solution (3µL) *per* condition, and eliminating the need for costly and specialized "angiogenesis plates" available from others, for example, Ibidi. Accordingly, Greiner LoBase plates were used for comparisons of the hydrogels' ability to support tubulogenesis, and to test the responsiveness of endothelial networks to the VEGF inhibitors Sunitinib and Axitinib. (Figure 4.)

To compare the capacity for tubulogenesis, a visible light cross-linked hydrogel that appeared comparable to the UV cross-linked hydrogels was identifed. The SPS:RuBPY₃ ratio was maintained and high (00) and low (--) concentration conditions were compared. The cell seeding density (1400-5500 cells/well) was also varied. The (--) condition produced improved endothelial cell attachment and more consistent endothelial networks than the (00) condition. The most consistent endothelial networks were observed between the 3900 and 5500 cells/well.

To quantitatively compare tubulogenesis a method of image analysis was generated wherein the images were optimized by creating a binary background subtraction, the binary was closed to eliminate artifacts from the binary mask creation, and then the networks were skeletonized. (Figure 5.) The total skeleton length and average length were then output to Excel for each image in order to compare total tubulogenesis per well, and characteristic features of the tubules (for example, whether there were either more or less tubules, or whether there were equivalent tubules, with one condition having tubules of longer length). This analysis method generated comparable results to an approach based on thresholding of individual images using previous data, and generating IC50s curves for Sunitinib inhibition of tubulogenesis. 384 well Greiner LoBase plates were used to generate IC50 as well as Z' scores for UV and visible light cross-linked gels with both HUVECs and iCell-ECs. With both photoinitiation schemes an increase in inhibitors decreased the observed total tubule length. (Figures 6, 7, 8 and 9.)

### Example 3- Thin hydrogel coatings

In this experimental example, very thin (10's to 100's of nm) hydrogels were directly conjugated to the surface of tissue culture polystyrene to take advantage of the observation that HUVEC seeding on non-treated tissue culture polystyrene resulted in transient and unstable endothelial networks that resembled tubules. In this approach the substrate was pegylated first, and substrate interactions are blocked thereby allowing only for cellular interactions. Then, increasing concentrations of cell adhesion peptides were added to tune the balance of cell-substrate interactions to stabilize tubules for an appropriate time frame before angiogenesis assays were performed (24-36 hrs). The thin hydrogel coatings of the present disclosure may be manufactured and sent as dry plate. RuBPY₃ photoinitiation was used because it is compatible with plate manufacturing in ambient light compared manufacturing that requires UV light exposure, and to take advantage of direct conjugation to phenolic residues on tissue culture-treated polystyrene.

Two options for direct conjugation with the thin hydrogel approach were tested *i.e.,* 1) using the phenolic residues for direct conjugation via RuBPY₃, or 2) first generating free thiols on the surface of the tissue culture treated polystyrene for cross-linking into the norborene containing hydrogel. To generate free thiols Traut's reagent was used in pH 9 water overnight at 37°C, and Ellman's assay was used to detect free thiols generated by this process. A dose dependent generation of free thiols on the polystyrene surface was observed, and the ability to modulate cell adhesion between non-thiol- and thiol-treated polystyrene substrates was compared. (Figure 10.) Both substrates were able to cross-link to the PEG in solution (Traut's treatment not shown), to block adhesion of HUVECs, and to mediate adhesion and tubule formation with increasing cell adhesion ligand present. Because both thiol-treated and non-thiol-treated substrates were comparable, Traut's reagent treatment was omitted as an added step that results in additional complexity and cost to downstream manufacturing.

To test the dependence of cross-linker degradability and concentrations of adhesion ligands for the preferred balance of cell-substrate interactions, the degradable peptide cross-linker that supported improved overall adhesion (monolayers forming at lower concentrations and a range of adhesion ligand concentrations to pursue for further resolution) was identified. To modulate cell-substrate interactions, the PEG:cross-linker ratio was varied. In addition to the PEG:cross-linker ratio influence on cell-substrate affinity, a higher ratio of PEG:cross-linker (60mg/mL to 4mM dithiol) provided the greatest tubulogenic activity, while lower concentrations of PEG resulted in greater monolayer formation. Inclusion of a degradable cross-linker improved the balance between monolayer and semi-stable tubule-like structures that remained attached at 24 hours. (Figures 12, 13 and 14.) A combination of 60mg/mL PEG-NB, 4mM dithiol cross-linker, 0.1mM cyclic RGD, and 0.8mM linear RGD was selected for further testing.

### Example 4 - Cell Encapsulation

A key advantage of visible light cross-linked hydrogels is that they avoid damaging UV light exposure to cells during encapsulation, or during overlay experiments. A hydrogel formulation was identified that provides a 3-dimensional vascular network that forms from cells encapsulated in the hydrogel. A 2x concentration of the hydrogel precursor was formulated to include 0.6 mg/mL RuBPY₃. iPSC-derived endothelial cells (Cellular Dynamics International) were trypsinized and harvested from their expansion cultures, and re-suspended in phosphate-buffered saline containing 1 mg/mL of sodium persulfate and 400 ng/mL vascular endotherlial growth factor (VEGF) (R&D systems). The cells were then added to the precursor solution at a 1:1 ratio, and 9 µL of this solution was pipetted into a well of an Ibid µ-slide Angiogenesis apparatus. The slide was exposed to blue LED illumination for 4 min. Fifty µL of PBS was added to the wells, aspirated, and an additional fifty µL of PBS was added to the wells. After a 10 minute incubation, the PBS was again aspirated and 50 uL of VascuLife VEGF complete medium (LifeLine Cell Technology), supplemented with the iCell Endothelial Cell Medium Supplement (Cellular Dynamics International) and 200 ng/mL VEGF was added to the wells. Medium was exchanged daily. Stable 3-D vascular networks form in the hydrogel. (Figure 15.)

### Example 5 - Overlay hydrogel

Overlay protocols with an extracellular matrix protein mixture (*i.e.,* Matrigel) have been shown to improve CYP450 assays using primary hepatocytes. In turn, there is interest in overlay protocols to enhance accumulation of deposited proteins including collagen in hepatocyte cultures, and the β-amyloid protein in neural cultures. Visible light cross-linking hydrogels are an alternative to the complex Matrigel material which may confound data analysis. Accordingly, HUVEC cells were plated onto a polymerized hydrogel that promotes tubule formation. The tubules are stable for 48 hours. At 24 hours after plating, the culture medium was removed and replaced with an overlay of a hydrogel precursor. The culture was then polymerized and maintained for an additional five days. The hydrogel overlay stabilized the tubules and allowed the tubules to mature into larger structures. (Figure 16.)

## Claims

1. A composition, comprising:
a) a visible light harvesting complex, wherein said visible light harvesting complex is RuBPY3;
b) a photoinitiator, wherein said photoinitiator is sodium persulfate (SPS);
c) a co-initiator, wherein said co-initiator is an aryl amine;
d) a di-thiol-terminated cross-linker; and
e) at least one cysteine-containing peptide.

2. The composition of claim 1, where said aryl amine is N-phenyl glycine.

3. The composition of claim 1, comprising:
a) 0 .03 mg/mL to 3.0 mg/mL RuBPY3;
b) 0.05 mg/mL to 5.0 mg/mL SPS; and
c) 0.1 mg/mL to 10.0 mg/mL N-phenyl glycine.

4. The composition of claim 1, further comprising at least one cell selected from the group consisting of a human umbilical vein endothelial cell, a human iPS-derived endothelial cell, a human iPS-derived retinal pigment epithelial cell, a human iPS-derived neuron, a human iPS-derived mesenchymal stem cell, a human iPS-derived photoreceptor cell, a human iPS-derived neural stem cell, a human iPS-derived microglia, and a human iPS-derived pericyte.

## Patentansprüche

1. Zusammensetzung, umfassend:
a) einen Sammelkomplex für sichtbares Licht, wobei es sich bei dem Sammelkomplex für sichtbares Licht um RuBPY3 handelt;
b) einen Photoinitiator, wobei es sich bei dem Photoinitiator um Natriumpersulfat (Sodium Persulfate, SPS) handelt;
c) einen Co-Initiator, wobei es sich bei dem Co-Initiator um ein Arylamin handelt;
d) einen dithiolterminierten Vernetzer; und
e) mindestens ein cysteinhaltiges Peptid.

2. Zusammensetzung nach Anspruch 1, wobei es sich bei dem Arylamin um N-Phenylglycin handelt.

3. Zusammensetzung nach Anspruch 1, umfassend:
a) 0,03 mg/ml bis 3,0 mg/ml RuBPY3;
b) 0,05 mg/ml bis 5,0 mg/ml SPS; und
c) 0,1 mg/ml bis 10,0 mg/ml N-Phenylglycin.

4. Zusammensetzung nach Anspruch 1, ferner umfassend mindestens eine Zelle, die aus der Gruppe bestehend aus einer menschlichen Nabelschnurvenenendothelzelle, einer menschlichen iPS-abgeleiteten Endothelzelle, einer menschlichen iPS-abgeleiteten Retinapigmentepithelzelle, einem menschlichen iPS-abgeleiteten Neuron, einer menschlichen iPS-abgeleiteten mesenchymalen Stammzelle, einer menschlichen iPS-abgeleiteten Photorezeptorzelle, einer menschlichen iPS-abgeleiteten neuronalen Stammzelle, einer menschlichen iPS-abgeleiteten Mikrogliazelle und einem menschlichen iPS-abgeleiteten Perizyten ausgewählt ist.

## Revendications

1. Composition, comprenant :
a) un complexe de collecte de lumière visible, ledit complexe de collecte de lumière visible étant RuBPY3 ;
b) un photoinitiateur, ledit photoinitiateur étant le persulfate de sodium (SPS) ;
c) un co-initiateur, ledit co-initiateur étant une arylamine ;
d) un agent de réticulation terminé par un di-thiol ; et
e) au moins un peptide contenant de la cystéine.

2. Composition selon la revendication 1, dans laquelle ladite arylamine est la N-phénylglycine.

3. Composition selon la revendication 1 comprenant :
a) 0,03 mg/mL à 3,0 mg/mL de RuBPY3 ;
b) 0,05 mg/mL à 5,0 mg/mL de SPS ; et
c) 0,1 mg/mL à 10,0 mg/mL de N-phénylglycine.

4. Composition selon la revendication 1, comprenant en outre au moins une cellule choisie dans le groupe constitué par une cellule endothéliale de veine ombilicale humaine, une cellule endothéliale dérivée d'iPS humaine, une cellule épithéliale pigmentaire rétinienne dérivée d'iPS humaine, un neurone dérivé d'iPS humaine, une cellule souche mésenchymateuse dérivée d'iPS humaine, une cellule photoréceptrice dérivée d'iPS humaine, une cellule souche neurale dérivée d'iPS humaine, une microglie dérivée d'iPS humaine et un péricyte dérivé d'iPS humaine.
